(19) Europäisches Patentamt
European Patent Office
Office européen des brevets

(11) Publication number: 0 092 986
A1

(12) **EUROPEAN PATENT APPLICATION**

(21) Application number: 83302287.4

(22) Date of filing: 22.04.83

(51) Int. Cl.³: C 07 D 499/68
A 61 K 31/43

(30) Priority: 23.04.82 GB 8211881

(43) Date of publication of application: 02.11.83 Bulletin 83/44

(84) Designated Contracting States: CH DE FR GB IT LI NL

(71) Applicant: BEECHAM GROUP PLC
Beecham House Great West Road
Brentford Middlesex(GB)

(72) Inventor: Hardy, Kenneth David
10 Oliver Road
Horsham Sussex(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Penicillins, a process for their preparation and compositions containing them.**

(57) A compound of formula (I) or a pharmaceutically acceptable salt or *in vivo* hydrolysable ester thereof:

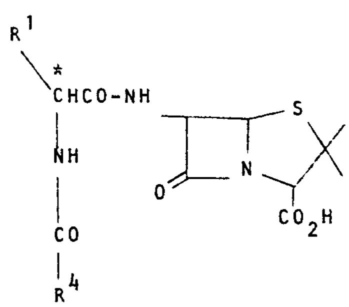

(I)

wherein $R^1$ represents a hydrocarbon or heterocyclyl group;

$R^4$ is a sub-group of formula (X) or (Y)

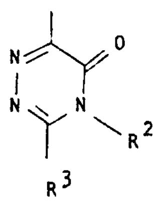

(X)

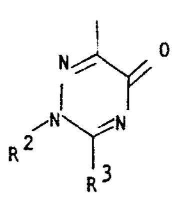

(Y)

wherein $R^2$ is hydrogen or $C_{1-6}$ alkyl; and $R^3$ is a sub-group of formula (A):

$$-S-R^5 \qquad (A)$$

wherein $R^5$ is an aryl group or a $C_{1-6}$ alkyl group substituted by an aryl group; or $R^3$ is a sub-group of formula (B):

$$-NH-R^6 \qquad (B)$$

wherein $R^6$ is an optionally substituted $C_{1-6}$ alkyl, aryl or cycloalkyl group; a process for the preparation of such compounds and compositions comprising them.

EP 0 092 986 A1

Croydon Printing Company Ltd.

RJB/B1239

0092986

# PENICILLINS, A PROCESS FOR THEIR PREPARATION AND COMPOSITIONS CONTAINING THEM

This invention relates to a class of penicillins which have antibacterial activity and are of value in the treatment of infections in animals, including mammals and especially humans. In particular the invention relates to a class of penicillins with the 1,2,4-triazine group in the side-chain. The invention also relates to a process for the preparation of such compounds, and to pharmaceutical compositions comprising them.

The present invention provides a compound of formula (I) or a pharmaceutically acceptable salt or *in vivo* hydrolysable ester thereof:

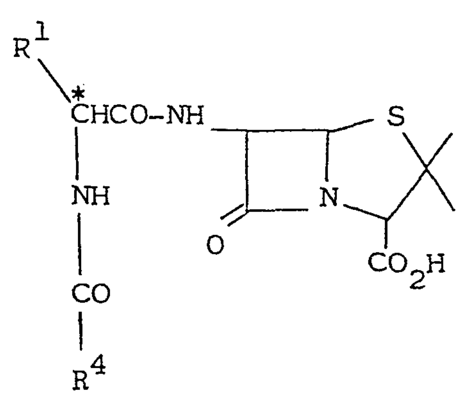

(I)

wherein $R^1$ represents a hydrocarbon or heterocyclyl group;

$R^4$ is a sub-group of formula (X) or (Y)

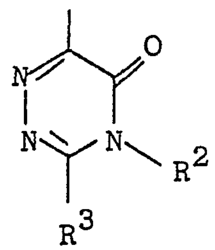

(X)

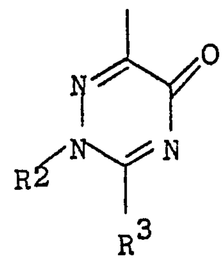

(Y)

wherein $R^2$ is hydrogen or $C_{1-6}$ alkyl; and $R^3$ is a sub-group of formula (A):

$$-S-R^5 \qquad (A)$$

wherein $R^5$ is an aryl group or a $C_{1-6}$ alkyl group substituted by an aryl group; or $R^3$ is a sub-group of formula (B):

$$-NH-R^6 \qquad (B)$$

wherein $R^6$ is an optionally substituted $C_{1-6}$ alkyl, aryl or cycloalkyl group.

Certain compounds within formula (I) may also occur in two or more tautomeric forms; these are also included within the scope of the present invention.

Examples of suitable pharmaceutically acceptable <u>in vivo</u> hydrolysable ester groups include those which break down readily in the human body to leave the parent acid or its salts, for example acyloxyalkyl groups, such as acetoxymethyl, pipaloyloxymethyl, α-acetoxyethyl and α-pivaloyloxyethyl groups;

alkoxycarbonyloxyalkyl groups, such as ethoxycarbonyloxymethyl and α-ethoxycarbonyloxyethyl; dialkylaminoalkyl groups, such as dimethylaminomethyl, dimethylaminoethyl, diethylaminomethyl or diethylaminoethyl; and lactone groups such as phthalidyl or dimethoxyphthalidyl.

Suitable pharmaceutically acceptable salts of the compounds of formula (I) include metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as dicyclohexylamine, or with procaine, dibenzylpiperidien, N-benzyl-β-phenethylamine, dehydroabietylamine, N,N'-bisdehydroabietylamine, ethylenediamine, or bases of the pyridine type such as pyridine, collidine or quinoline.

The carbon atom marked * in formula (I) is asymmetric and the compound may be derived from the side-chain having a D, L or DL configuration at that position. All forms of compound (I) are included in this invention. Suitably, the carbon atom marked * is derived from the D-configuration and is conveniently referred to as the D-penicillin.

The term 'hydrocarbon' includes groups having up to 18 carbon atoms, suitably up to 10 carbon atoms, conveniently up to 6 carbon atoms. Suitable hydrocarbon groups include $C_{1-6}$ alkyl, $C_{2-6}$ alkenyl, $C_{2-6}$ alkynyl, $C_{3-7}$ cycloalkyl, $C_{3-7}$ cycloalkyl($C_{1-6}$)-alkyl, aryl, and aryl($C_{1-6}$)alkyl.

When used herein the term 'aryl' includes phenyl and naphthyl optionally substituted with up to five halogen, $C_{1-6}$ alkyl, $C_{1-6}$ alkoxy, halo $(C_{1-6})$ alkyl, hydroxy, amino, carboxy, $C_{1-6}$ alkoxycarbonyl, or $C_{1-6}$ alkoxycarbonyl-$(C_{1-6})$-alkyl groups.

The term 'heterocyclyl' includes single or fused rings comprising up to four hetero atoms in the ring selected from oxygen, nitrogen and sulphur and optionally substituted with up to three halogen, $C_{1-6}$ alkyl, $C_{1-6}$ alkoxy, halo-$(C_{1-6})$-alkyl, hydroxy, amino, carboxy, $C_{1-6}$ alkoxycarbonyl, $C_{1-6}$ alkoxycarbonyl$(C_{1-6})$ alkyl, aryl or oxo groups.

Suitably $R^1$ in formula (I) is phenyl, 4-hydroxyphenyl, or a 5- or 6- membered heterocyclic ring containing up to three heteroatoms selected from oxygen, sulphur or nitrogen, optionally substituted with hydroxy, amino, halogen or $C_{1-6}$ alkoxy.

Suitably $R^1$ is phenyl, 4-hydroxyphenyl, 2-thienyl, 3-thienyl or 2-amino-4-thiazolyl.

Preferably $R^1$ is phenyl or 4-hydroxyphenyl, especially phenyl.

Preferably $R^2$ is hydrogen.

Suitable groups $R^6$ include $C_{1-6}$ alkyl optionally substituted by halogen, carboxy, $C_{1-6}$ alkoxycarbonyl, carbamoyl, aryl, heterocyclyl, hydroxy, $C_{1-6}$ alkanoyloxy, amino, mono- and di- $(C_{1-6})$ alkylamino, aryl, $C_{1-6}$ alkylthio, $C_{1-6}$alkyloxy and $C_{3-7}$ cycloalkyl.

Preferably $R^6$ represents phenyl or $C_{1-4}$ alkyl.

When used herein the term 'lower' refers to groups of up to six carbon atoms.

Suitable $C_{1-6}$ alkyl groups for $R^2$, $R^5$ and $R^6$ may be straight or branched chain and include methyl, ethyl n- or iso-propyl, n-, sec-, iso- or tert-butyl. In those cases where the $C_{1-6}$ alkyl group carries a substituent the preferred $C_{1-6}$ alkyl groups for $R^5$ and $R^6$ include methyl, ethyl and n-propyl.

Preferred values of $R^3$ within the present invention are those of formula (B) and include methylamino, ethylamino, butylamino, anilino, and 4-aminosulphonylphenylamino.

Preferably $R^3$ is anilino or butylamino.

Particular compounds within formula (I) include:
6β-[D,2-(3-benzylamino-1,2,4-triazine-5-one-6-carbonylamino)-2-phenyl]acetamido penicillanic acid;
6β-[D,2-(3-anilino-1,2,4-triazine-5-one-6-carbonylamino)-2-phenyl]acetamido penicillanic acid;
6β-[D,2-(3-butylamino-1,2,4-triazine-5-one-6-carbonylamino)-2-phenyl]acetamido penicillanic acid.

The compounds of formula (I) may be prepared by reacting a compound of formula (II):

(II)

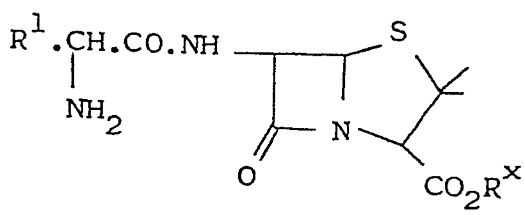

wherein the amino group is optionally substituted with a group which permits acylation to take place, $R^1$ is as defined with respect to formula (I) and any reactive substituents may be protected, and $R^x$ is hydrogen or a carboxyl-blocking group, with an N-acylating derivative of an acid of formula (III):

$$\begin{array}{l} CO_2H \\ | \\ R^4 \end{array} \qquad (III)$$

wherein $R^4$ is as defined with respect to formula (I) above and any reactive groups may be protected; and thereafter, if necessary, carrying out one or more of the following steps:

(i) removing any carboxyl-blocking groups $R^x$;

(ii) removing any protecting groups on the side chain group;

(iii) converting the product into a salt or in vivo hydrolysable ester thereof.

Suitable groups which permit acylation to take place and which are optionally present on the amino group of the starting material of the formula (II) include N-silyl, N-stannyl and N-phosphorus groups, for example trialkylsilyl groups such as trimethylsilyl, trialkyltin groups such as tri-*n*-butyltin, groups of formula $-P.R^aR^b$ wherein $R^a$ is an alkyl, haloalkyl, aryl, aralkyl, alkoxy, haloalkyl, aryl, aralkyl, alkoxy, haloalkoxy, aryloxy, aralkyloxy or dialkylamino group, $R^b$ is the same as $R^a$ or is halogen or $R^a$ and $R^b$ together form a ring; suitable such phosphorus groups being $-P(OC_2H_5)_2$, $-P(C_2H_5)_2$,

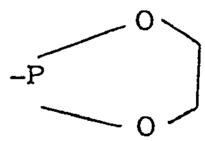

and

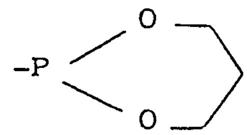

Suitable carboxyl-blocking derivatives for the group $-CO_2R^x$ in formula (II) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with trilower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, such as described above, an oxime radical of formula $-N=CHR^o$ where $R^o$ is aryl or heterocyclic, or an *in vivo* hydrolysable ester radical such as defined above.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

A reactive N-acylating derivative of the acid (III) is employed in the above process. The choice of reactive derivative will of course be influenced by the chemical nature of the substituents of the acid.

Suitable N-acylating derivatives include an acid halide, preferably the acid chloride or bromide. Acylation with an acid halide may be affected in the presence of an acid binding agent for example, tertiary amine (such as triethylamine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2,alkylene oxide - such as ethylene oxide or propylene oxide. The acylation reaction using an acid halide may be carried out at a temperature in th range -50°C to +50°C, preferably -20°C to +20°C, in aqueous or non-aqueous media such as aqueous acetone, aqueous tetrahydroform, ethyl, acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (III) or a salt thereof with a halogenating (eg chlorinating or brominating) agent such as phosphorus pentachloride, thionyl chloride or oxalyl chloride.

Alternatively, the N-acylating derivative of the acid (III) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids

(such as phosphoric or phosphorous acids) or aliphatic or aromatic sulphonic acids (such as p-toluenesulphonic acid). When a symmetrical anhydride is employed, the reaction may be carried out in the presence of 2,6-lutidine as catalyst.

Alternative N-acylating derivatives of acid (III) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, or 8-hydroxyquinoline; or amides such as N-acylsaccharins or N-acylphthalimides; or an alkylidene iminoester prepared by reaction of the acid (III) with an oxime.

Other reactive N-acylating derivatives of the acid (III) include the reactive intermediates formed by reaction in situ with a condensing agent such as a carbodiimide, for example, N,N-diethyl-, dipropyl- or diisopropylcarbodiimide, N,N'-di-cyclohexylcarbodiimide, or N-ethyl-N'-γ-dimethylaminopropylcarbodiimide; a suitable carbonyl compound, for example, N,N'-carbonyldiimidazole or N,N'-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example $BBr_3 - C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

The intermediate compound of formula (II) may be prepared by reacting a compound of formula (V):

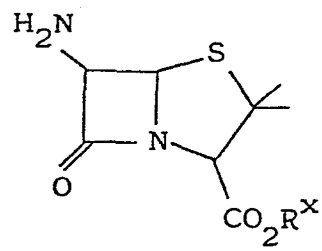

(V):

wherein the amino group is optionally substituted with a group which permits acylation to take place and $R^x$ is as defined with respect to formula (II) above, with an N-acylating derivative of an acid of formula (VI):

$$\underset{NHR^y}{R^1.CH.CO_2H} \qquad (VI)$$

wherein $R^1$ is as defined with respect to formula (I) and any reactive groups therein may be protected and $R^y$ is an amino-protecting group; and thereafter removing protecting group $R^y$.

Suitable N-acylating derivatives, carboxyl protecting groups and reaction conditions include those described hereinbefore.

Suitable amino-protecting groups $R^y$ are those well-known in the art which may be removed under conventional conditions without disruption of the remainder of the molecule.

The compounds of formula (I) may also be prepared by reacting a compound of formula (V) as described hereinbefore with an N-acylating derivative of an acid of formula (VII):

$R^1.CH.CO_2H$
|
NH
|
CO
|
$R^4$

(VII)

wherein $R^1$ and $R^4$ are as defined with respect to formula (I) and any reactive groups therein may be protected; and thereafter, if necessary, carrying out one or more of the following steps:

i) removing any carboxyl-blocking group $R^x$

ii) removing any protecting groups on the side-chain group;

iii) converting the product into a salt or in vivo hydrolysable ester thereof.

Compounds within formula (I) wherein $R^4$ is a sub-group of formula (B) may also be prepared by reacting a compound of formula (VIII):

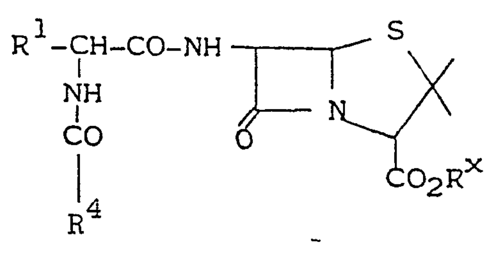

(VIII)

wherein $R^1$, $R^4$ and $R^x$ are as hereinbefore defined and wherein $R^3$ is $SR^5$; any reactive groups within compounds of formula (VIII) may be protected, with a compound of formula (IX):

$R^6-NH_2$ (IX)

wherein $R^6$ is as hereinbefore defined; and thereafter, if necessary, carrying out one or more of the following steps:

i) removing any carboxyl-blocking group $R^x$

ii) removing any protecting groups on the side-chain group;

iii) converting the product into a salt or in vivo hydrolysable ester thereof.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The composition may be formulated for administration by any route, such as oral topical or parenteral. The compositions may be in the form of tablets, capsules, powders, granules, lozenges, creams or liquid preparations, such as oral or sterile parenteral solutions or suspensions.

Tablets and capsules for administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica;

disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water

removed under vacuum. The dry lyophilized powder is then sealed in the vial and an accompanying vial of water for injection may be supplied to reconstitute the liquid prior to use. Parental suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 300 mg per day, for instance 1500 mg per day depending on the route and frequency of administration.

The compound of formula (I) may be the sole therapeutic agent in the compositions of the invention or a combination with other antibiotics or with a β-lactamase inhibitor may be employed.

Advantageously, the compositions also comprise a compound of formula (X) or a pharmaceutically acceptable salt or ester thereof:

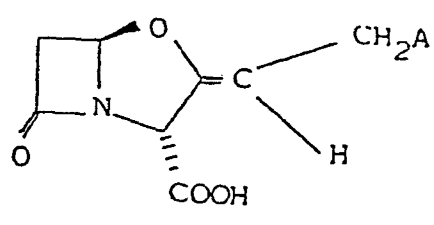

(X)

wherein A is hydroxyl, substituted hydroxyl, thiol, substituted thiol, amino, mono- or di-hydrocarbyl-substituted amino, or mono- or di-acylamino.

A further advantageous composition comprises a compound of formula (I) or a pharamceutically acceptable salt or in vivo hydrolysable ester thereof together with a compound of formula (XI) or a pharmaceutically acceptable salt or in vivo hydrolysable ester thereof:

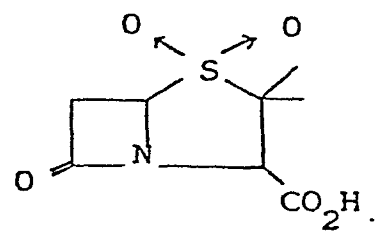

(XI)

The present invention also provides a method of treating bacterial infections in animals, in particular humans or domestic mammals, which comprises the administration of a composition of this invention

The following Examples illustrate the preparation of the compounds of this invention.

Example 1

6β-[D,2-(3-Anilino-1,2,4-triazine-5-one-6-carbonylamino)-2-phenyl]acetamido penicillanic acid;

3-Anilino-5-hydroxy-1,2,4-triazine-6-carboxylic acid (0.47g 0.002 mol) dissolved in methylene dichloride (20 ml) and triethylamine (1.5ml) was cooled to -20°C and treated with thionyl chloride (0.15). The solution was stirred at -20°C for 1 hr and treated all at once with a solution of anhydrous ampicillin (0.7g. 0.002 mol) in methylene dichloride (20 ml) and triethylamine (0.6ml) cooled to 0°C. The reaction solution was stirred at RT for 1hr and evaporated. The residue, dissolved in water (30 ml) was washed with ether (2 x 30 ml), covered with ethylacetate (30 ml) and acidified with shaking, to pH 1.5 with 5N hydrochloric acid. The insoluble material was separated, dissolved in methanol (20 ml) treated with 2N sodium 2-ethyl hexanoate in methyl isobutyl ketone (1 ml) and the solution evaporated to small volume. The residual oil was diluted with excess dry ether and the separated solid filtered washed well with dry ether and dried *in vacuo* to give the required penicillin sodium salt 0.39g (34.2%). δ[$(CD_3)_2$ $SO/D_2O$] 1.46 and 1.60 (6H, 2s, gemdimethyls), 4.11 (1H, s, $C_3$ proton), 5.40 (2H, ABq., β-lactam protons), 6.02 (1H, s, α-proton), 7.00-8.00 (5H, m, anilino protons) 7.40 (5H, m, phenyl protons). Rf (BEW) 0.35.

Example 2

6β-[D,2-(3-Butylamino-1,2,4-triazine-5-one-6-carbonylamino)-2-phenyl]acetamido penicillanic acid.

3-n-Butylamino-5-hydroxy-1,2,4-triazine-6-carboxylic acid (0.26g 0.0012mol) dissolved in methylene dichloride (20 ml) and triethylamine (1ml) was cooled to -20°C and treated with thionyl chloride (0.11ml). The solution was stirred at -20°C and treated all at once with a solution of anhydrous ampicillin (0.42g 0.0012mol) in methylene dichloride (20 ml) and triethylamine (0.4ml) cooled to 0°C. The solution was stirred at RT for 1hr and evaporated to dryness. The residue dissolved in water (20 ml) was washed with ether (2 x 20ml) covered with ethylacetate (20 ml) and acidified, with shaking, to pH 1.5 with 5N hydrochloric acid. The aqueous layer was re-extracted with ethyl acetate (20ml) and the combined organic extracts dried over anhydrous magnesium sulphate. The dry solution was treated with 2N sodium 2-ethyl hexanoate in methyl isobutyl ketone (0.7ml). The separated solid was filtered, washed well with dry ether and dried *in vacuo* to give the required penicillin sodium salt 0.45g (66.4%). $\delta[(CD_3)_2SO/D_2O]$ 0.84 (3H, t, $NHCH_2(CH_2)_2\underline{CH_3}$), 1.20 (4H, m, $CH_2(\underline{CH_2})_2CH_3$), 1.45 and 1.57 (6H, 2s, gemdimethyls), 3.28 (2H, t, $NH\underline{CH_2}$), 4.06 (1H, s, $C_3$ proton), 5.46 (2H, q, β-lactam protons), 5.97 (1H, s, -proton), 7.47 (5H, m, aromatic protons) RF (BEW) 0.40.

BIOLOGICAL DATA

| ORGANISM | Compound of Example No. | |
| --- | --- | --- |
| | 1 | 2 |
| E.coli ESS | 0.2 | 0.1 |
| E. coli NCTC 10418 | 12.5 | 5.0 |
| Ps. aeruginosa NCTC 10662 $10^{-2}$ | 12.5 | 5.0 |
| P mirabilis C977 | 12.5 | 2.5 |
| B subtilis | 0.5 | 0.5 |
| S aureus Oxford | 0.1 | 0.5 |
| N. catarrhalis 1502 | 0.02 | 0.02 |
| S. faecalis I | 5.0 | 12.5 |
| S. pyogenes CN 10 | 1.2 | 0.05 |

0092986

A

CLAIMS

1 A compound of formula (I) or a pharmaceutically acceptable salt or in vivo hydrolysable ester thereof:

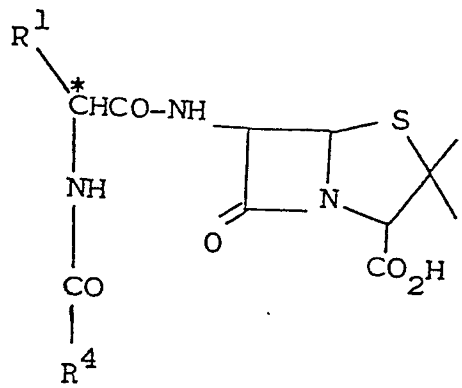

(I)

wherein $R^1$ represents a hydrocarbon or heterocyclyl group;

$R^4$ is a sub-group of formula (X) or (Y)

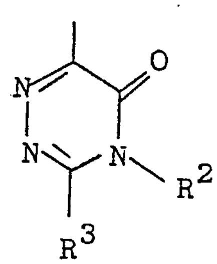

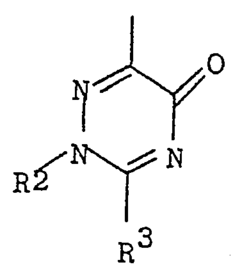

(X) (Y)

wherein $R^2$ is hydrogen or $C_{1-6}$ alkyl; and $R^3$ is a sub-group of formula (A):

$$-S-R^5 \qquad (A)$$

wherein $R^5$ is an aryl group or a $C_{1-6}$ alkyl group substituted by an aryl group; or $R^3$ is a sub-group of formula (B):

$$-NH-R^6 \qquad (B)$$

wherein $R^6$ is an optionally substituted $C_{1-6}$ alkyl, aryl or cycloalkyl group.

2 A compound as claimed in claim 1 wherein $R^1$ is phenyl, 4-hydroxyphenyl, or a 5- or 6- membered heterocyclic ring containing up to three heteroatoms selected from oxygen, sulphur or nitrogen, optionally substituted with hydroxy, amino, halogen or $C_{1-6}$ alkoxy.

3 A compound as claimed in either of claims 1 or 2 wherein $R^6$ is phenyl or $C_{1-4}$ alkyl.

4 A compound as claimed in any of claims 1 - 3 wherein $R^2$ is hydrogen.

5 6β-[2-(3-Benzylamino-1,2,4-triazine-5-one-6-carbonylamino)-2-phenyl]acetamido penicillanic acid;
6β-[2-(3-anilino-1,2,4-triazine-5-one-6-carbonylamino)-2-phenyl]acetamido penicillanic acid;
6β-[2-(3-butylamino-1,2,4-triazine-5-one-6-carbonylamino)-2-phenyl]acetamido penicillanic acid or pharmaceutically acceptable salts or in vivo hydrolysable esters thereof.

6 A process for the preparation of a compound as claimed in claim 1, which process comprises:

A) reacting a compound of formula (II):

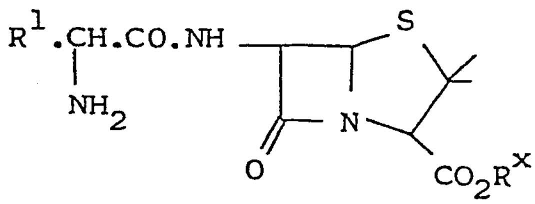

wherein the amino group is optionally substituted with a group which permits acylation to take place, $R^1$ is as defined with respect to formula (I) and any reactive substituents may be protected, and $R^x$ is hydrogen or a carboxyl-blocking group, with an N-acylating derivative of an acid of formula (III):

$CO_2H$

|

$R^4$ (III)

wherein $R^4$ is as defined with respect to formula (I) above and any reactive groups may be protected;

B) by reacting a compound of formula (V):

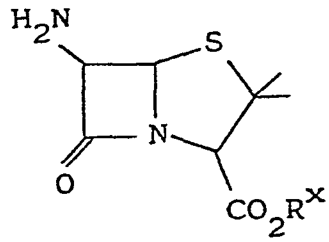

(V)

wherein the amino group is optionally substituted with a group which permits acylation to take place and $R^x$ is as defined with respect to formula (II) above, with an N-acylating derivative of an acid of formula (VII):

$R^1$.CH.$CO_2H$
|
NH
|
CO
|
$R^4$

(VII)

wherein $R^1$ and $R^4$ are as defined with respect to formula (I) and any reactive groups therein may be protected; and after steps A or B, if necessary carrying out one or more of the following steps:

i) removing any carboxyl-blocking group $R^x$

ii) removing any protecting groups on the side-chain group;

iii) converting the product into a salt or in vivo hydrolysable ester thereof.

7 A process for the preparation of compounds claimed in claim (I) wherein $R^4$ is of the sub-group of formula (B), which process comprises reacting a compound of formula (VIII):

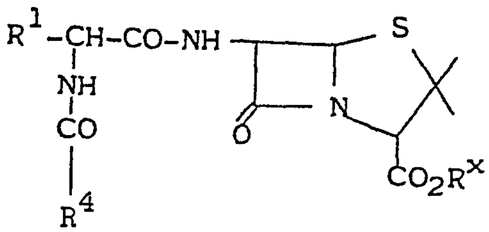

(VIII)

wherein $R^1$, $R^4$ and $R^x$ are as hereinbefore defined and wherein $R^3$ is $SR^5$; any reactive groups within compounds of formula (VIII) may be protected, with a compound of formula (IX):

$$R^6-NH_2 \qquad (IX)$$

wherein $R^6$ is as hereinbefore defined; and thereafter, if necessary, carrying out one or more of the following steps:

i) removing any carboxyl-blocking group $R^x$

ii) removing any protecting groups on the side-chain group;

iii) converting the product into a salt or in vivo hydrolysable ester thereof.

8 A composition which comprises a compound as claimed in any one of claims 1 to 5 together with a pharmaceutically acceptable carrier or excipient.

9 A composition as claimed in claim 8 which also comprises a β-lactamase inhibitor.

10 A compound as claimed in any of claims 1 to 5 for use in the treatment of antibacterial infections in the human or animal body.

0092986

Application number

European Patent Office

# PARTIAL EUROPEAN SEARCH REPORT

which under Rule 45 of the European Patent Convention shall be considered, for the purposes of subsequent proceedings, as the European search report

EP 83 30 2287

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US - A - 4 081 441 (S.R. BAKER)<br>* column 7, lines 44-68; column 10, lines 1-26,42-64; claims *<br>-- | 1,6,8 | C 07 D 499/68<br>A 61 K 31/43 |
| Y | CHEMICAL ABSTRACTS, vol.92, no.7, February 18, 1980, page 675, abstract no.58771t COLUMBUS OHIO (US) & JP - A - 79 106 489 (TOYO JOZO CO., LTD.)(21-08-1979)<br>* abstract *<br>-- | 1,6,8 | |
| A | US - A - 4 005 075 (H. YAMADA)<br>* column 2, lines 30-32; columns 3,4; claims 1,4; abstract *<br>--<br>./. | 1,6,8 | TECHNICAL FIELDS SEARCHED (Int. Cl. 3)<br>C 07 D 499/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9

Claims searched incompletely:

Claims not searched: 10

Reason for the limitation of the search:

Method for treatment of the human or animal body by surgery or therapy (see article 52(4) of the European Patent Convention).

| Place of search | Date of completion of the search | Examiner |
|---|---|---|
| The Hague | 01-07-1983 | CHOULY |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82'

European Patent Office

**PARTIAL EUROPEAN SEARCH REPORT**

0092986

Application number

EP 83 30 2287 -2-

| DOCUMENTS CONSIDERED TO BE RELEVANT | | | CLASSIFICATION OF THE APPLICATION (Int Cl.3) |
|---|---|---|---|
| Category | Citation of document with indication, where appropriate, of relevant passages | Relevant to claim | |
| A | CHEMICAL ABSTRACTS, vol.80, no.7, February 18, 1974, page 299, abstract no.37122u COLUMBUS OHIO (US) & JP - A - 73 72 189 (YAMANOUCHI PHARMACEUTICAL CO., LTD.)(29-09-1973)<br>* abstract *<br>--------- | 1,6,8 | |
| | | | TECHNICAL FIELDS SEARCHED (Int. Cl.3) |